# EUROPÄISCHE PATENTSCHRIFT

(11) **EP 2 550 066 B1**
(45) Veröffentlichungstag und Bekanntmachung des Hinweises auf die Patenterteilung: **28.06.2017**
(21) Anmeldenummer: 11701143.7
(22) Anmeldetag: 28.01.2011
(51) Int. Cl.: A61Q 5/10, A61K 8/37, A61K 8/31, A61K 8/03, A61K 8/81

(54) **VISKOSITÄTSGEBENDE ZWEIPHASEN-ENTWICKLER MIT KLARER ÖLPHASE FÜR OXIDATIVE FARBVERÄNDERUNGSMITTEL**
VISCOSITY-PRODUCING TWO PHASE DEVELOPER WITH A CLEAR OIL PHASE FOR OXIDATIVE COLOUR MODIFYING AGENTS
RÉVÉLATEUR DIPHASIQUE PROMOTEUR DE VISCOSITÉ, AYANT UNE PHASE HUILEUSE CLAIRE, POUR DES PRODUITS MODIFICATEURS DE COULEUR PAR OXYDATION

(30) Priorität: 25.03.2010 DE 102010003264
(43) Veröffentlichungstag der Anmeldung: 30.01.2013
(73) Patentinhaber: Henkel AG & Co. KGaA, 40589 Düsseldorf (DE)
(72) Erfinder: GOUTSIS, Konstantin, 41812 Erkelenz (DE); JANßEN, Frank, 41470 Neuss (DE); WADLE, Armin, 40699 Erkrath (DE); KRIPPAHL, Marc, 40789 Monheim (DE)
(86) Internationale Anmeldenummer: PCT/EP2011/051193
(87) Internationale Veröffentlichungsnummer: WO 2011/117004

(56) Entgegenhaltungen:
- EP-A1- 0 308 825
- EP-A1- 1 438 951
- WO-A2-2011/082910

## Beschreibung

Gegenstand der vorliegenden Anmeldung ist eine kosmetische Zubereitung für keratinische Fasern, insbesondere menschliche Haare, welche sich durch zwei voneinander getrennte Phasen auszeichnet, wobei eine der Phasen eine wässrige Phase und die andere Phase eine klare, hydrophobe Ölphase darstellt. Diese Zubereitungen enthalten zumindest ein chemisches Oxidationsmittel, mindestens ein anionisches, polymeres Verdickungsmittel und weiterhin mindestens ein Carbonsäureesteröl und/oder mindestens ein Paraffinöl. In ihrer Eigenschaft als oxidierende Zubereitungen werden diese Mittel als Entwicklerzubereitung für Oxidationsfärbungen oder Aufhellmittel eingesetzt. Ein weiterer Gegenstand der vorliegenden Erfindung ist daher ein Verfahren zur Farbveränderung keratinischer Fasern, bei dem die erfindungsgemäßen Mittel zur Farbveränderung auf keratinische Fasern aufgebracht werden.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln oder von Luftsauerstoff untereinander die eigentlichen Farbstoffe ausbilden. Die Oxidationsfärbemittel zeichnen sich durch hervorragende, lang anhaltende Färbeergebnisse aus. Für temporäre Färbungen werden üblicherweise Färbe- oder Tönungsmittel verwendet, die als färbende Komponente sogenannte direktziehende Farbstoffe ("Direktzieher") enthalten. Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Sollen Substrate aufgehellt oder gar gebleicht werden, werden die das Substrat färbenden Farbstoffe meist oxidativ unter Einsatz von entsprechenden Oxidationsmitteln, wie Wasserstoffperoxid, entfärbt.

Um eine optimale Färbeleistung zu entfalten, benötigen oxidative Färbemittel in der Regel einen alkalischen pH-Wert zur Ausfärbung, insbesondere zwischen pH 9,0 und pH 10,5. Darüber hinaus beträgt die Anwendungsdauer für ansprechende Färbeergebnisse üblicherweise zwischen 10 und 45 min. Es ist daher notwendig, dass das anwendungsbereite Färbemittel so formuliert und konfektioniert ist, dass das Färbemittel sich einerseits gut auf den zu färbenden keratinischen Fasern verteilen lässt, andererseits jedoch in den zu färbenden Fasern während der Anwendungszeit verbleibt. Dazu ist es vorteilhaft, wenn das Färbemittel über eine bestimmte Viskosität verfügt, die zwar das Auftragen des Mittels ermöglicht, jedoch das Mittel auch am Ort der Anwendung verbleiben lässt. Diese Viskosität kann durch polymere Verdickungsmittel im anwendungsbereiten Färbemittel eingestellt werden, wobei dieses Verdickungsmittel sowohl in der Farbveränderungszubereitung oder der Oxidationsmittelzubereitung enthalten sein kann.

Um eine gute Mischung von Farbveränderungszubereitung und Oxidationsmittelzubereitung zu ermöglichen, ist es vorteilhaft, wenn die Farbveränderungszubereitung und Oxidationsmittelzubereitung über eine gute Fließfähigkeit verfügen und sich die erhöhte Viskosität der Anwendungsmischung erst nach Vermischen der beiden Komponenten einstellt. Gängige polymere Verdickungsmitteln in kosmetischen Zubereitungen sind Derivate von Cellulosen, wie Hydroxyethylcellulose oder Xanthan. Eine weitere Möglichkeit, dieses Ziel zu erreichen, ist der Einsatz von polymeren Verdickungsmitteln, deren verdickende Eigenschaften sich mit dem pH-Wert ändern. Die Farbveränderungszubereitung besitzt zur Stabilisierung von Oxidationsfarbstoffvorprodukten zumeist einen alkalischen pH-Wert und die Oxidationsmittelzubereitung besitzt zur Stabilisierung des Oxidationsmittels einen sauren pH-Wert, während die Anwendungsmischung einen alkalischen pH-Wert besitzen sollte.

Wenn der polymere Verdicker in der sauren Oxidationsmittelzubereitung enthalten ist, ist ein anionischer polymerer Verdicker bevorzugt, der bei einem alkalischen pH-Wert zu einer deutlichen Viskositätserhöhung führt. Als solche anionischen, polymeren Verdickungsmittel eignen sich besonders Homo- oder Copolymere von Acrylsäure oder Methacrylsäure.

Neben der Viskositätseinstellung der Anwendungszubereitung sollen die Oxidationsmittelzubereitungen auch über pflegende Eigenschaften verfügen und diese durch ein geeignetes optisches, kosmetisches Erscheinungsbild verdeutlichen. Dazu sind insbesondere zweiphasige Oxidationsmittelzubereitungen geeignet, die neben einer wässrigen, oxidationsmittelhaltigen Phase eine weitere pflegende, hydrophobe Phase enthalten. Dabei ist es aus Stabilitätsgründen unerlässlich, dass die beiden Phasen zweiphasigen Oxidationsmittelzubereitungen klar und scharf voneinander getrennt vorliegen und dass keine Zwischenphasen oder Mischphasen im Phasengrenzbereich auftreten. Auch sollen Eintrübungen der Phasen durch Schlierenbildungen oder partiellen Emulsionsbereichen in den Phasen vermieden werden. Es ist daher von besonderem Vorteil, wenn insbesondere die hydrophobe Ölphase klar und transparent vorliegt. Unter "klar" und "transparent" ist hierbei eine Transmission von wenigstens 60%, bevorzugt wenigstens 80% zu verstehen. Diese Transmission wird mit UV/VIS-Spectrometrie gemessen. Die Transmission kann dabei bei unterschiedlichen Wellenlängen gemessen werden, bevorzugt bei Raumtemperatur, bei einer Wellenlänge von λ = 600 nm und einer Küvettenlänge von 10 mm.

Zweiphasige Pflegezubereitungen sind aus WO2006/042174 A1 bekannt. Darin werden zwei stabile Phasen beschrieben, die in Kontakt miteinander verpackt werden, wobei eine Phase eine schäumende Reinigungsphase und eine weitere Phase eine nichtschäumende, wässrige Phase darstellt. Aus der WO2006/042174 A1 sind keine zweiphasigen Mittel mit hydrophober und hydrophiler Phase bekannt. EP 308825A1 offenbart emulsionsförmige Wasserstoffperoxid-Zubereitungen zum Blondieren und oxidativen Färben der Haare wobei der Begriff "emulsionsförmig" eine permanente Emulsion mit einer diskontinuierlichen Öl- oder Fettphase und einer kontinuierlichen Wasserphase bezeichnet.
Aufgabe der vorliegenden Erfindung ist es daher, ein farbveränderndes Mittel zur Verfügung zu stellen, durch welches die oben genannten Nachteile gebräuchlicher farbverändernden Mittel herabgesenkt werden. Insbesondere ist es die Aufgabe der vorliegenden Erfindung, eine zweiphasige Oxidationszubereitung für oxidative Farbveränderungsmittel von keratinische Fasern zur Verfügung zu stellen, welche eine deutliche Phasengrenze zwischen hydrophober Ölphase und wässriger Phase besitzt, eine klare, nicht eingetrübte hydrophobe Ölphase enthält und zu einer guten Mischbarkeit mit der Farbveränderungszubereitung führt. Dabei soll die resultierende Anwendungsmischung jedoch eine ausreichende Viskosität aufweist, so dass das Mittel sich einerseits gut auftragen lässt, andererseits während der Anwendung am Wirkort verbleibt und nicht aus den Fasern ausfließt.
In nicht vorhersehbarer Weise konnte nun gefunden werden, dass in farbverändernden Mitteln für keratinische Fasern, insbesondere menschliche Haare, diese Aufgaben durch spezielle zweiphasige, oxidative Zubereitungen, die neben einem Oxidationsmittel bestimmte anionische, polymere Verdickungsmittel sowie ein hydrophobes Öl enthalten, in besonderer Weise gelöst werden. Ein erster Gegenstand der vorliegenden Erfindung ist daher ein kosmetisches Mittel für die Behandlung keratinischer Fasern, das dadurch gekennzeichnet ist, dass es zwei nebeneinander vorliegende, aber durch eine Phasengrenze voneinander getrennte Phasen umfasst, die in zwei Schichten übereinander mit unmittelbarem Kontakt über eine gemeinsame Grenzfläche zueinander vorliegen,
wobei die erste Phase (I) eine wässrige Phase darstellt, die Wasserstoffperoxid und mindestens ein anionisches, polymeres Verdickungsmittel, ausgewählt aus vernetzten oder unvernetzten Polyacrylsäure-Polymeren enthält, und
wobei die zweite Phase (II) eine klare, hydrophobe Ölphase darstellt, die als Öl mindestens einen flüssigen Carbonsäureester aus C₂-C₈-Monoalkanol mit einer Mono- oder Dicarbonsäure und/oder mindestens ein Paraffinöl enthält, dadurch gekennzeichnet, dass das anionische polymere Verdickungsmittel ausgewählt ist aus Polyacrylsäuren, die ein Molekulargewicht MW von mindestens 500000 g/mol besitzen, das Mittel nichtionische, anionische, zwitterionische und/oder amphotere Tenside und/oder Emulgatoren in einem Gesamtgewicht von weniger als 1 Gew.-%, jeweils bezogen auf das Gesamtgewicht des Mittels, enthält und das Öl der klaren, hydrophoben Ölphase (II) ausgewählt ist aus der Gruppe, die gebildet wird aus Isopropylpalmitat, Isopropylmyristat, Dibutyladipat und flüssigem Paraffinöl.
Unter keratinhaltigen bzw. keratinischen Fasern werden erfindungsgemäß Pelze, Wolle, Federn und insbesondere menschliche Haare verstanden. Obwohl die erfindungsgemäße Verwendung in erster Linie zum Färben und/oder Aufhellen von keratinhaltigen Fasern geeignet sind, steht prinzipiell einer Verwendung auch auf anderen Gebieten nichts entgegen.
Entscheidendes Merkmal der erfindungsgemäßen Zubereitung ist dabei die Zweiphasigkeit, wobei die beiden Phasen nicht miteinander mischbar sind. Vorzugsweise liegen die beiden Phasen in zwei Schichten übereinander mit unmittelbarem Kontakt über eine gemeinsame Grenzfläche zueinander vor.

In der erfindungsgemäßen Zubereitung liegt die Phase (I) bevorzugt zu mindestens gleichen Gewichtsanteil wie die Phase (II) vor. Bevorzugt liegt Phase (I) im Überschuss vor. Bevorzugt besitzt das Gewichtsverhältnis von Phase (I) zu Phase (II) einen Wert von 99 zu 1 bis 50 zu 50, bevorzugt von 98 zu 2 bis 70 zu 30, besonders bevorzugt von 95 zu 5 bis 80 zu 20.

Gemäß der vorliegenden Erfindung besitzt die erste Phase (I) einen wässrigen oder wässrig-alkoholischen Träger. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Ein wässriger Träger enthält im Sinne der Erfindung mindestens 30 Gew.-%, insbesondere mindestens 50 Gew.-% Wasser, bezogen auf das Gesamtgewicht der Anwendungsmischung.
Weiterhin enthält die erste Phase (I) Wasserstoffperoxid als chemisches Oxidationsmittel. Der Begriff "chemisches Oxidationsmittel" soll dabei verdeutlichen, dass es sich dabei um ein extra zugesetztes Oxidationsmittel handelt und nicht etwa um ein in der Umgebung vorliegendes Oxidationsmittel, wie beispielsweise Luftsauerstoff. Die Konzentration einer Wasserstoffperoxid-Lösung wird einerseits von den gesetzlichen Vorgaben und andererseits von dem gewünschten Effekt bestimmt. Vorzugsweise werden als wässrige Phase 3 Gew.-%ige bis 12 Gew.-%ige Lösungen in Wasser verwendet.
Die erfindungsgemäßen Zubereitungen enthalten Wasserstoffperoxid. Hier sind erfindungsgemäße Mittel zur Farbveränderung keratinischer Fasern besonders bevorzugt, die 0,5 bis 18 Gew.-%, vorzugsweise 1 bis 15 Gew.-%, besonders bevorzugt 2,5 bis 12 Gew.-% und insbesondere 3 bis 9 Gew.-% Wasserstoffperoxid (berechnet als 100%iges H₂O₂) enthalten.

Die wässrige Phase (I) enthält weiterhin als anionisches, polymeres Verdickungsmittel mindestens ein vernetztes oder unvernetztes Polyacrylsäure-Polymer, das ausgewählt ist aus Polyacrylsäuren, die ein Molekulargewicht MW von mindestens 500000 g/mol besitzen.

Unter vernetzten Polyacrylsäure-Polymeren sind solche Polymerisate zu verstehen, denen zur Vernetzung während der Polymerisation ein gewisser Anteil an bi- oder multifunktionalen Monomeren zugesetzt wurde. Bevorzugte Vernetzungsmittel sind dabei Ethylenglycoldimethacrylat, 1,9-Decadien, Divinylbenzol sowie Allylether von Pentaerythrit, von Sucrose, von Ethylenglycol und von Propylenglycol. Geeignete polymere Verbindungen sind beispielsweise unter dem Warenzeichnen Carbopol^{®} im Handel erhältlich und unter der INCI-Bezeichnung Carbomer bekannt.

Das erfindungsgemäße anionische, polymere Verdickungsmittel weist ein mittleres Molekulargewicht von mindestens 500.000 g/mol auf, um einerseits eine ausreichende Verdickung in der Anwendungsmischung zu erzielen und andererseits eine hinreichende Löslichkeit in der wässrigen Phase (I) zu gewährleisten.

Ein erfindungsgemäß besonders bevorzugtes anionisches, polymeres Verdickungsmittel wird unter dem Handelsnamen Carbomer 954 von der Firma Lubrizol vertrieben.

Bevorzugt enthält das erfindungsgemäße Mittel das oder die anionische(n), polymere(n) Verdickungsmittel zu 0,01 bis 20 Gew.-%, bevorzugt zu 0,1 bis 5 Gew.-%, jeweils bezogen auf das Gesamtgewicht des Mittels.

Gemäß der vorliegenden Erfindung ist die zweite Phase (II) hydrophober Natur. Die erfindungsgemäße hydrophobe Phase (II) ist mit der wässrigen Phase (I), enthaltend das Oxidationsmittel, nicht mischbar. Hydrophobe Phasen, auch lipophile Phasen genannt, enthalten Fettkörper, die üblicherweise unpolare organische Verbindungen wie Kohlenwasserstoffverbindungen, langkettige Triglyceride, Siliconöle, Ester oder Ether sowie perhalogenierte Verbindungen enthalten.

Entscheidend für die vorliegende Erfindung ist dabei, dass die Ölphase klar und transparent vorliegt. Das bedeutet, dass sie keine Eintrübungen, Schlieren oder Mikroemulsionen, insbesondere im Bereich der Grenzfläche zur hydrophilen Phase, aufweisen soll.

Die klare, hydrophobe Ölphase (II) der vorliegenden Erfindung zeichnet sich dadurch aus, dass sie mindestens einen flüssigen Carbonsäureester aus C₂-C₈-Monoalkanol mit einer Mono- oder Dicarbonsäure, ausgewählt aus Isopropylpalmitat, Isopropylmyristat und Dibutyladipat, und/oder mindestens ein flüssiges Paraffinöl enthält. Der Begriff "flüssig" bezeichnet dabei auf bei Raumtemperatur und unter Normaldruck flüssige Carbonsäureester und/oder Paraffinöle. Erfindungsgemäße geeignete Carbonsäureester sind solche, die keine oder nur eine sehr geringe Wasserlöslichkeit besitzen, d. h. eine Wasserlöslichkeit von unter 1 g pro 1 L Wasser bei Normalbedingungen.

Paraffinöle sind nicht wasserlöslich und eignen sich daher ebenfalls als hydrophobe Phase (II) in den erfindungsgemäßen zweiphasigen Mitteln. Unter den Paraffinölen der hydrophoben Phase sind Gemische gesättigter, aliphatischer Kohlenwasserstoffe zu verstehen, welche bei Raumtemperatur flüssig sind. Erfindungsgemäß bevorzugte Mittel enthalten als hydrophobe Phase (II) daher zumindest ein flüssiges Paraffinöl.

Bevorzugt enthält das Mittel des ersten Erfindungsgegenstands als Öl der klaren, hydrophoben Ölphase (II) 1 bis 80 Gew.-%, bevorzugt 2 bis 50 Gew.-% und insbesondere bevorzugt 5 bis 30 Gew.-%, jeweils bezogen auf das Gesamtgewicht des Mittels, an Isopropylpalmitat und/oder Isopropylmyristat und/oder Dibutyladipat und/oder flüssigem Paraffinöl sowie gegebenenfalls weiterer unpolarer organischer Verbindungen wie Kohlenwasserstoffverbindungen, langkettige Triglyceride, Siliconöle, Ester oder Ether sowie perhalogenierte Verbindungen.

Besonders bevorzugt enthält die klare, hydrophobe Ölphase (II) zumindest
zu 5 bis 30 Gew.-% Isopropylmyristat oder
zu 5 bis 30 Gew.-% Isopropylpalmitat oder
zu 5 bis 30 Gew.-% Dibutyladipat oder
zu 5 bis 30 Gew.-% flüssiges Paraffinöl.

Um die Trennung von hydrophiler Phase (I) und hydrophober Phase (II) zu verbessern und eine klare, hydrophobe Phase (II) zu erhalten, muss die Neigung des Mittels zur Bildung einer stabilen Emulsion reduziert sein. Daher ist es erfindungsgemäß bevorzugt, wenn das Mittel nur einen geringen Anteil an grenzflächenaktiven Substanzen enthält. Als grenzflächenaktive Substanzen im Sinne der Erfindung gelten dabei Emulgatoren und Tenside. Grenzflächenaktive Substanzen zeichnen sich durch hydrophobe und hydrophile Strukturmerkmale aus und ermöglichen so eine Durchmischung der Phasen unter Ausbildung von Micellen und stabilen Emulsionen. Da die vorliegende Erfindung explizit keine Emulsionen umfasst, sondern vielmehr zwei getrennt voneinander vorliegende Phasen enthält, hat es sich als erfindungsgemäß notwendig herausgestellt, dass das Mittel nichtionische, anionische, zwitterionische und/oder amphotere Tenside und/oder Emulgatoren in einem Gesamtgewicht von weniger als 1 Gew.-%, jeweils bezogen auf das Gesamtgewicht des Mittels, enthält. Besonders vorteilhaft sind Mittel, die frei sind von grenzflächenaktiven Substanzen.

Anionische Tenside im Sinne der Erfindung sind alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für solche anionischen Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren mit 8 bis 30 C-Atomen (Seifen); Ethercarbonsäuren, insbesondere der Formel RO(CH₂CH₂O)ₓCH₂COOH, in der R eine lineare Alkylgruppe mit 8 bis 30 C-Atomen und x = 0 oder 1 bis 16 ist; Acylsarcoside; Acyltauride; Acylisethionate; Sulfobernsteinsäuremono- und -dialkylester sowie Sulfobernsteinsäuremono-alkylpolyoxyethylester; lineare Alkansulfonate; lineare α-Olefinsulfonate; Sulfonate ungesättigter Fettsäuren; α-Sulfofettsäuremethylester von Fettsäuren; Alkylsulfate und Alkylethersulfate, insbesondere der Formel RO(CH₂CH₂O)ₓSO₃H, in der R für eine lineare Alkylgruppe mit 8 bis 30 C-Atomen und x für 0 oder eine Zahl von 1 bis 12 steht; Gemische oberflächenaktiver Hydroxysulfonate; sulfatierte Hydroxyalkylpolyethylen- und/oder Hydroxyalkylenpropylenglykolether; Ester der Weinsäure und Zitronensäure mit Alkoholen; Alkyl- und/oder Alkenyletherphosphate der Formel RO(C₂H₄O)ₓP(=O)(OH)(OR'), worin R für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht; sulfatierte Fettsäurealkylenglykolester der Formel RC(O)O(alkO)ₙSO₃H, in der R für einen linearen oder verzweigten, aliphatischen, gesättigten und/oder ungesättigten Alkylrest mit 6 bis 22 C-Atomen, alk für CH₂CH₂, CHCH₃CH₂ und/oder CH₂CHCH₃ und n für eine Zahl von 0,5 bis 5 steht; sowie Monoglyceridsulfate und Monoglyceridethersulfate.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Beispiele solcher zwitterionischen Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Übliche amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Beispielhafte amphotere Tenside sind N-Kokosalkylaminopropionat, Kokosacylaminoethylaminopropionat und C₁₂-C₁₈-Acylsarcosin.

Nichtionische Tenside und Emulgatoren enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe; mit einem Methyl- oder C₂-C₆-Alkylrest endgruppenverschlossene Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol LS, Dehydol LT (Cognis) erhältlichen Typen; Polyglycerinester und alkoxylierte Polyglycerinester, wie beispielsweise Poly(3)glycerindiisostearat (Handelsprodukt: Lameform TGI (Henkel)) und Poly(2)glycerinpolyhydroxy-stearat (Handelsprodukt: Dehymuls PGPH (Henkel)); Polyolfettsäureester, wie beispielsweise das Handelsprodukt Hydagen HSP (Cognis) oder Sovermol-Typen (Cognis); höher alkoxylierte, propoxylierte und insbesondere ethoxylierte, Mono-, Di- und Triglyceride mit Alkoxylierungsgrad von größer als 5, wie beispielsweise Glycerinmonolaurat + 20 Ethylenoxid und Glycerinmonostearat + 20 Ethylenoxid; Aminoxide; Hydroxymischether; Sorbitanfettsäureester und Anlagerungeprodukte von Ethylenoxid an Sorbitanfettsäureester wie beispielsweise die Polysorbate und Sorbitanmonolaurat + 20 Mol Ethylenoxid (EO); Zuckerfettsäureester und Anlagerungsprodukte von Ethylenoxid an Zuckerfettsäureester; Anlagerungsprodukte von Ethylenoxid an Fettsäurealkanolamide und Fettamine; Fettsäure-N-alkylglucamide; Alkylphenole und Alkylphenolalkoxylate mit 6 bis 21, insbesondere 6 bis 15 Kohlenstoffatomen in der Alkylkette und 5 bis 30 Ethylenoxid- und/oder Propylenoxid-Einheiten; Alkylpolyglykoside entsprechend der allgemeinen Formel RO-(Z)ₓ, wobei R für Alkyl, Z für Zucker sowie x für die Anzahl der Zuckereinheiten steht.

Zu den nichtionischen Emulgatoren im Sinne der Erfindung zählen weiterhin die Polymerisationsprodukte aus Ethylenoxid und Propylenoxid an gesättigte oder ungesättigte Fettalkohole; Fettsäureester mehrwertiger Alkohole mit gesättigten oder ungesättigten Fettsäuren; Alkylester von gesättigten oder ungesättigten Fettsäuren oder Alkylphenole und deren Alkoxylate; insbesondere Ethylenglykolether von Fettalkoholen; gemischte Ethylen- und Propylenglykolether mit Fettalkoholen; Fettsäureester an Sorbitan sowie Polyethylenglykol; Ester von nicht hydroxylierten C₆-C₃₀-Alkylmonocarbonäuren mit Polyethylenglykol; und Anlagerungsprodukte von Alkylphenolen an Ethylen- und/oder Propylenoxid.

Weiterhin kann es zur Trennung der hydrophilen und hydrophoben Phase im erfindungsgemäßen Mittel vorteilhaft sein, zusätzlich dem Mittel Elektrolyte zuzugeben. Unter Elektrolyten werden üblicherweise geladene, ionische anorganische und organische Verbindungen verstanden, welche über keinen oder nur einen nur sehr gering ausgeprägten hydrophoben Anteil enthalten. Bevorzugte Elektrolyte sind gut wasserlösliche Salze, insbesondere Alkalimetall- und Erdalkalimetallsalze von Mineralsäuren und organischen Säuren. Beispiele hierfür sind Natriumchlorid, Natriumsulfat, Natriumhydrogensulfat, Natriumcarbonat, Natriumhydrogencarbonat, Natriumcitrat, Magnesiumchlorid, Magnesiumsulfat, Magnesiumcarbonat und Magnesiumhydrogencarbonat.

Das erfindungsgemäße Mittel zeichnet sich dadurch aus, dass sich öllösliche Inhaltsstoffe überwiegend in der hydrophoben Phase (II) anreichern und daher nicht in unmittelbaren Kontakt mit der Oxidationsmittel-haltigen Phase (I) kommen. Dies ist besonders vorteilhaft, um oxidativ wenig stabile Pflegestoffe im Mittel zu stabilisieren. Solche bevorzugte Pflegestoffe sind daher öllösliche Pflegestoffe, öllösliche Vitamine und Triglyceride, insbesondere pflanzliche und solche, die eine oder mehrere ungesättigte Kohlenstoff-Kohlenstoff-Bindungen enthalten. Um die Zweiphasigkeit optisch zu verdeutlichen, kann es ebenso sinnvoll sein, wenn die hydrophobe Phase (II) öllösliche Farbstoffe enthält.

Eine besondere Ausführungsform der vorliegenden Erfindung ist daher dadurch gekennzeichnet, dass das Mittel zusätzlich mindestens eine überwiegend öllösliche Komponente, ausgewählt aus öllöslichen Farbstoffen, öllöslichen Pflegestoffen, öllöslichen Vitaminen und Triglyceriden, enthält.

Als überwiegend öllöslich werden erfindungsgemäß solche Verbindungen bezeichnet, die eine Wasserlöslichkeit von unter 1 g pro 1 L Wasser bei Normalbedingungen besitzen, jedoch in apolaren Verbindungen gut (d.h. > 10 g / kg Lösungsmedium) löslich sind.

Öllösliche Pflegestoffe sind beispielsweise kosmetisch wirksame Terpene und Terpenoide, wie beispielsweise Bisabolol, und Ubichinone, wie beispielsweise Coenzym Q-10.

Öllösliche Vitamine sind insbesondere die Verbindungen, die unter den Sammelbezeichnungen Vitamin A, Vitamin D, Vitamin E und Vitamin K bekannt sind. Ein erfindungsgemäß bevorzugtes Mittel enthält daher mindestens ein öllösliches Vitamin, ausgewählt aus Vitamin A, Vitamin D, Vitamin E und/oder Vitamin K sowie Vitamin P. Vitamin A umfasst dabei Retinoide, insbesondere *all-trans-*Retinol. Vitamin D, auch als Calciferole bezeichnet, umfasst 7,8-Didehydrosterol-Derivate, insbesondere die Verbindungen mit der Bezeichnung Cholecalciferol (Vitamin D₃, Calciol), Ergocalciferol (Vitamin D₂, Ercalciol), 7,8-Didehydrocholesterol (Provitamin D₃, Procalciol, Procholecalciferol) und Ergosterol (Provitamin D₂). Weitere, einsetzbare Vitamin D-Analoga sind Calcidiol (25-Hydroxycholecalciferol), Calcitriol, Hydroxycalcidiol und Vitamin D₁ (Ergocalciferol und Lumisterol). Vitamin E ist die Sammelbezeichnung für Tocopherole und umfasst insbesondere die chemischen Verbindungen α-Tocopherol, β-Tocopherol, γ-Tocopherol, δ-Tocopherol und α-Tocotrienol, β-Tocotrienol, γ-Tocotrienol und δ-Tocotrienol. Vitamin K ist Sammelbezeichnung für verschiedene Verbindungen mit Vitamin-K-Aktivität, die sich von 2-Methyl-1,4-naphthochinon (Vitamin K₃) ableiten. Bevorzugte Vertreter sind Vitamin K₁₍₂₀₎ (2-Methyl-3-phytyl-1,4-naphthochinon), Phyllochinon (Kurzzeichen: K),], Vitamin K₂₍₃₅₎ (3-all-trans-Farnesylgeranylgeranyl-2-methyl-1,4-naphthochinon), Vitamin K₃ (2-Methyl-1,4-naphthochinon, Menadion, Menaphthon) sowie die abgeleiteten Analoga Vitamin K₄ (2-Methyl-1,4-naphthalindiol), Vitamin K₅ (4-Amino-2-methyl-1-naphthol), Vitamin K₆ (2-Methyl-1,4-naphthalindiamin) und Vitamin K₇ (4-Amino-3-methyl-1-naphthol). Bei Vitamin P handelt es sich um eine Sammelbezeichnung für Rutine, insbesondere Bioflavonoide wie Troxerutin (Vitamin P₄) und Hesperidin.

Triglyceride sind die Sammelbezeichnung für Ester des Glycerins, welche die Hauptbestandteile natürlicher Öle darstellen. Erfindungsgemäß besonders bevorzugte Triglyceride sind solche, die zumindest einen Ester einer ungesättigten Fettsäure enthalten. Bevorzugte ungesättigte Fettsäuren sind Ölsäure, Linolsäure und Linolensäure. Weiterhin können als Triglyceride bevorzugt pflanzliche Öle eingesetzt werden, insbesondere solche, die einen positiven Einfluss auf die Haaroberfläche besitzen. Besonders geeignete Triglyceride sind dabei insbesondere Öle, die aus den Saaten von Moringa pterygosperma (Moringaöl) oder aus den Kernen von Argania Spinosa (Arganöl) gewonnen werden. Diese Öle werden beispielsweise unter der Bezeichnung Lipofructyl® ARGAN LS 9779 bzw. Lipofructyl® MO LS 9305 von der Firma Cognis vertrieben.

Eine Ausführungsform des ersten Erfindungsgegenstands ist daher dadurch gekennzeichnet, dass die die hydrophobe Phase (II) zusätzlich mindestens ein Öl enthält, welches ausgewählt ist aus Ölen aus den Saaten von Moringa pterygosperma (Moringaöl) und/oder aus den Kernen von Argania Spinosa (Arganöl).

Bevorzugt werden die überwiegend öllöslichen Komponenten in einem Gesamtgewicht von 0,001 bis 10 Gew.-%, insbesondere von 0,01 bis 5 Gew.-%, jeweils bezogen auf das Gesamtgewicht der hydrophoben Phase (II), eingesetzt.

Die erfindungsgemäßen Mittel dienen bevorzugt zur Farbveränderung keratinischer Fasern. Dazu wird das erfindungsgemäße, zweiphasige Mittel (M1) mit einem weiteren Mittel (M2), enthaltend mindestens eine farbverändernde Komponente, vermischt und die resultierende, anwendungsbereite Zubereitung auf die keratinische Fasern gegeben.

Die erfindungsgemäßen Mittel (M1) weisen dabei, insbesondere zur Stabilisierung der Oxidationsmittel, bevorzugt einen sauren pH-Wert im Bereich von 2,5 bis 6 auf. Bei den pH-Werten im Sinne der vorliegenden Erfindung handelt es sich um pH-Werte, die bei einer Temperatur von 22 °C gemessen wurden.

Als farbverändernde Komponente in Mittel (M2) dienen als Aufhellmittel zusätzliche Bleichkraftverstärker, die die Wirkung des Oxidationsmittels aus Phase (I) des zweiphasigen Mittels verstärken, sowie farbgebende Komponenten.

In einer Ausführungsform enthält das erfindungsgemäße Mittel (M2) daher einen zusätzlichen Bleichkraftverstärker. Als zusätzliche Bleichkraftverstärker können im Rahmen dieser Erfindung Peroxoverbindungen, weiterhin Verbindungen, die unter Perhydrolysebedingungen aliphatische Peroxocarbonsäuren und/oder substituierte Perbenzoesäure ergeben, Kohlensäurederivate, Alkylcarbonate, -carbamate, Silylcarbonate und -carbamate eingesetzt werden.

Vorzugsweise ist der Bleichkraftverstärker ausgewählt aus Ammoniumperoxodisulfat, Alkalimetallperoxodisulfaten, Ammoniumperoxomonosulfat, Alkalimetallhydrogenperoxomonosulfaten, Alkalimetallperoxodiphosphaten und Erdalkalimetallperoxiden. Besonders bevorzugte Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid. Erfindungsgemäß besonders bevorzugt sind Mittel, die als Bleichkraftverstärker mindestens ein anorganisches Salz, ausgewählt aus Peroxomonosulfaten und/oder Peroxodisulfaten, enthalten.

Weiter hin hat es sich bei den Arbeiten zur vorliegenden Erfindung als besonders bevorzugt erwiesen, wenn die erfindungsgemäßen Mittel mindestens zwei verschiedene Peroxodisulfate enthalten. Bevorzugte Peroxodisulfatsalze sind dabei Kombinationen aus Ammoniumperoxodisulfat und Kaliumperoxodisulfat und/oder Natriumperoxodisulfat. Die Peroxoverbindungen sind in einer Menge von 0,1 bis 25 Gew.-%, insbesondere in einer Menge von 0,5 bis 15 Gew.-%, bezogen auf das Gesamtgewicht des anwendungsbereiten Mittels, enthalten.

Der Einsatz von Persulfatsalzen bzw. Peroxodisulfatsalzen erfolgt in der Regel wasserfrei und in Form eines gegebenenfalls entstaubten Pulvers, Paste oder eines in Form gepressten Formkörpers. Die wasserfreien Mittel (M2) können anstelle und/oder zusätzlich zu den festen Peroxoverbindungen einen weiteren Bleichkraftverstärker enthalten.

Obwohl prinzipiell keine Einschränkungen hinsichtlich der Formulierung der weiteren Mittel (M2) bestehen, hat es sich erfindungsgemäß als bevorzugt erwiesen, wenn Mittel (M2) wasserfrei formuliert ist. Wasserfrei im Sinne der vorliegenden Erfindung bedeutet einen Wassergehalt bezogen auf die Mittel (M2) von weniger als 5 Gew.-%, insbesondere von weniger als 2 Gew.-%. Blondierzubereitungen, die weniger als 0,1 Gew.-% Wasser, enthalten können erfindungsgemäß besonders bevorzugt sein. Mittel (M2) ist vorzugsweise als Pulver oder als wasserfreie Paste formuliert.

In einer weiteren, bevorzugten Ausführungsform kann das Mittel (M2) als Bleichkraftverstärker mindestens ein kationisches Pyridinium-Derivat enthalten. Bevorzugte Verbindungen sind 4-AcylPyridinium-Derivate und 2-Acylpyridinium-Derivate. Insbesondere bevorzugt sind dabei 2-Acetyl-1-methylpyridinium-p-toluolsulfonat und 4-Acetyl-1-methylpyridinium-p-toluolsulfonat. Weiterhin bevorzugte kationische Pyridinium-Derivate sind dabei kationische 3,4-Dihydroisochinolinium-Derivat. Insbesondere bevorzugt ist N-Methyl-3,4-dihydroisochinolinium-p-toluolsulfonat.

Die neben oder anstelle von Peroxoverbindungen eingesetzten Bleichkraftverstärker sind in den erfindungsgemäßen, kosmetischen Mitteln bevorzugt in Mengen von 0,05 bis 10 Gew.-%, insbesondere in Mengen von 0,2 bis 5 Gew.-%, jeweils bezogen auf das Gesamtgewicht des anwendungsbereiten Mittels, enthalten.

Zur weiteren Steigerung der Aufhellleistung können der erfindungsgemäßen Zusammensetzung zusätzlich als Bleichverstärker mindestens eine gegebenenfalls hydratisierte SiO₂-Verbindung zugesetzt. Obwohl bereits geringe Mengen der gegebenenfalls hydratisierten SiO₂-Verbindungen die Aufhellleistung erhöhen, kann es erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder. Bevorzugte gegebenenfalls hydratisierten SiO₂-Verbindungen sind Kieselsäuren, deren Oligomeren und Polymeren sowie deren Salze. Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder besonders bevorzugt als Wasserglas eingesetzt. Erfindungsgemäß bevorzugt sind Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt. Insbesondere Metasilicate, die sich gemäß vorstehender Formel durch das Verhältnis zwischen n und der Summe aus m und p von ≤ 1 auszeichnen und sich als kettenförmige polymere Strukturen des Anions [SiO_{3]}²⁻ auffassen lassen, können bevorzugt eingesetzt werden. Natriummetasilicat der Formel [NaSiO₃]ₓ, ist dabei besonders bevorzugt.

In einer weiteren Ausführungsform der vorliegenden Erfindung enthält das Mittel (M2) als farbverändernde Komponente farbgebende Komponenten. Die erfindungsgemäßen Mittel können daher zusätzlich mindestens eine farbgebende Komponente enthalten, die bevorzugt ausgewählt wird aus mindestens einem Oxidationsfarbstoffvorprodukt und/oder aus mindestens einem direktziehenden Farbstoff.

Erfindungsgemäß bevorzugte Mittel zum Farbveränderung keratinischer Fasern sind demnach dadurch gekennzeichnet, dass sie mindestens ein Oxidationsfarbstoffvorprodukt enthalten. Die erfindungsgemäßen Aufhellmittel enthalten als Oxidationsfarbstoffvorprodukt mindestens ein Oxidationsfarbstoffvorprodukt vom Entwicklertyp (Entwicklerkomponente), bevorzugt in Kombination mit mindestens einer Oxidationsfarbstoffvorprodukt vom Kupplertyp (Kupplerkomponente).

Bevorzugte Oxidationsfarbstoffvorprodukte vom Entwicklertyp sind p-Phenylendiaminderivate. Bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Di-aminobenzo-1,4-dioxan sowie ihren physiologisch verträglichen Salzen. Erfindungsgemäß besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Di-hydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie deren physiologisch verträglichen Salzen. Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Ganz besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines ihrer physiologisch verträglichen Salze. Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)-phenol, 4-Amino-2-(1,2-dihydroxyethyl)-phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Ganz besonders bevorzugte Verbindungen sind p-Aminophenol, 4-Amino-3-methylphenol, 4-Amino-2-aminomethylphenol, 4-Amino-2-(1,2-dihydroxyethyl)-phenol und 4-Amino-2-(diethylaminomethyl)phenol. Ferner kann die Entwicklerkomponente ausgewählt sein aus o-Aminophenol und seinen Derivaten, wie 2-Amino-4-methylphenol, 2-Amino-5-methylphenol oder 2-Amino-4-chlorphenol. Weiterhin kann die Entwicklerkomponente ausgewählt sein aus heterocyclischen Entwicklerkomponenten, wie Pyrimidinderivaten, Pyrazolderivaten, Pyrazolopyrimidin-und Pyrazolopyrazol-Derivaten bzw. ihren physiologisch verträglichen Salzen. Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-tri-aminopyrimidin, 2,4-Dihydroxy-5,6-diaminopyrimidin und 2,5,6-Triaminopyrimidin. Bevorzugte Pyrazol-Derivate sind insbesondere die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze, insbesondere jedoch 4,5-Diamino-1-(2-hydroxyethyl)pyrazol. Bevorzugte Pyrazolopyrimidine sind die Verbindungen, die ausgewählt werden unter Pyrazolo[1,5-a]pyrimidin-3,7-diamin, 2,5-Dimethyl-pyrazolo[1,5-a]pyrimidin-3,7-diamin, Pyrazolo-[1,5-a]pyrimidin-3,5-diamin, 2,7-Dimethyl-pyrazolo[1,5-a]pyrimidin-3,5-diamin, 3-Aminopyrazolo-[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo-[1,5-a]pyrimidin-7-yl)-(2-hydroxy-ethyl)-amino]ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen, wenn ein tautomeres Gleichgewicht vorhanden ist. Bevorzugtes Pyrazolopyrazol-Derivat ist 2,3-Diamino-6,7-dihydro-1H,5H-pyrazolo[1,2-a]pyrazol-1-on.

Besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen. Ganz besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1 H-imidazol-1-yl)propyl]amin, und/oder 4,5-Diamino-1-(2-hydroxyethyl)-pyrazol sowie deren physiologisch verträglichen Salze.

Die Entwicklerkomponenten werden bevorzugt in einer Menge von 0,0001 bis 0,5 Gew.-%, vorzugsweise 0,001 bis 0,2 Gew.-%, jeweils bezogen auf das anwendungsbereite Mittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt. Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus.

Erfindungsgemäße Kupplerkomponenten werden bevorzugt als mindestens eine Verbindung aus einer der folgenden Klassen ausgewählt: m-Aminophenol, o-Aminophenol, m-Diaminobenzol, o-Diaminobenzol und/oder deren Derivate; Naphthalinderivate mit mindestens einer Hydroxygruppe; Di- beziehungsweise Trihydroxybenzol; Pyridinderivate; Pyrimidinderivate; bestimmte IndolDerivate und Indolin-Derivate; Pyrazolonderivate (beispielsweise 1-Phenyl-3-methylpyrazol-5-on); Morpholinderivate (beispielsweise 6-Hydroxybenzomorpholin oder 6-Aminobenzomorpholin); Chinoxalinderivate (beispielsweise 6-Methyl-1,2,3,4-tetrahydrochinoxalin), sowie Gemische aus zwei oder mehreren Verbindungen aus einer oder mehreren dieser Klassen.

Bevorzugte m-Aminophenol-Kupplerkomponenten werden ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)benzol, 3-Ethylamino-4-methylphenol, 2,4-Dichlor-3-aminophenol und deren physiologisch verträglichen Salzen. Bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis-(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen. Bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und deren physiologisch verträglichen Salzen. Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin. Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol. Bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)-amino-3-amino-6-methoxypyridin, 2-(4'-Methoxyphenyl)amino-3-aminopyridin, und deren physiologisch verträglichen Salzen. Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen. Bevorzugte Indolderivate werden ausgewählt aus mindestens einer Verbindung der Gruppe, die gebildet wird aus 4-Hydroxyindol, 6-Hydroxyindol und 7-Hydroxyindol und deren physiologisch verträglichen Salzen. Bevorzugte Indolinderivate werden ausgewählt aus mindestens einer Verbindung der Gruppe, die gebildet wird aus 4-Hydroxyindolin, 6-Hydroxyindolin und 7-Hydroxyindolin und deren physiologisch verträglichen Salzen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxy-ethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxy-benzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Ganz besonders bevorzugt sind Resorcin, 2-Methylresorcin, 5-Amino-2-methylphenol, 3-Aminophenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,0001 bis 0,5 Gew.-%, vorzugsweise 0,001 bis 0,2 Gew.-%, jeweils bezogen auf das anwendungsbereite Mittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Weiterhin können die erfindungsgemäßen Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe können in anionische, kationische und nichtionische direktziehende Farbstoffe unterteilt werden. Üblicherweise sind es Nitrophenylendiamine, Nitroaminophenole, Azofarbstoffe, Anthrachinone oder Indophenole. Die direktziehenden Farbstoffe werden jeweils bevorzugt in einer Menge von 0,0001 bis 0,2 Gew.-%, bevorzugt von 0,001 bis 0,1 Gew.-%, jeweils bezogen auf die gesamte Anwendungszubereitung, eingesetzt. Die Gesamtmenge an direktziehenden Farbstoffen beträgt vorzugsweise höchstens 0,1 Gew.-%.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Bevorzugte kationische direktziehende Farbstoffe sind kationische Triphenylmethanfarbstoffe, wie Basic Blue 7, Basic Blue 26, Basic Violet 2 und Basic Violet 14, aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor^{®} vertrieben werden, sind erfindungsgemäß ebenfalls besonders bevorzugte kationische direktziehende Farbstoffe. Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)-amino-4-nitrobenzol, 2-[(4-Amino-2-nitrophenyl)amino]-benzoesäure, 6-Nitro-1,2,3,4-tetrahydro-chinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol. Erfindungsgemäß bevorzugte Farbstoffkombinationen sind solche, die mindestens die Kombination aus Tetrabromphenolblau und Acid Red 92; Tetrabromphenolblau und Acid Red 98; Tetrabromphenolblau und Acid Red 94; Tetrabromphenolblau und Acid Red 87 oder Tetrabromphenolblau und Acid Red 51.

Erfindungsgemäße, anwendungsbereite Mittel sind vorzugsweise wässrige, fließfähige Zubereitungen. Die erfindungsgemäßen Mittel können weiterhin alle für solche Zubereitungen bekannten Wirk-, Zusatz- und Hilfsstoffe enthalten. Die anwendungsbereiten Mittel als Mischung aus Mittel (M1) und (M2) können dabei oberflächenaktive Substanzen, ausgewählt aus den oben angeführten anionischen, nichtionischen, zwitterionischen und amphoteren Tensiden enthalten.

Erfindungsgemäß bevorzugt sind in anwendungsbereiten Mitteln kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Alkylamidoamine werden üblicherweise durch Amidierung natürlicher oder synthetischer Fettsäuren und Fettsäureschnitte mit Dialkylaminoaminen hergestellt, wie Stearamidopropyl-dimethylamin. Ebenfalls bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex, Dehyquart und Armocare vertrieben. Die Produkte Armocare VGH-70, ein N,N-Bis(2-Palmitoyloxyethyl)dimethylammoniumchlorid, sowie Dehyquart F-75, Dehyquart C-4046, Dehyquart L80 und Dehyquart AU-35 sind Beispiele für solche Esterquats. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können nicht-ionische, zwitterionische und/oder amphotere Tenside sowie deren Mischungen bevorzugt sein.

Erfindungsgemäß kann aber die Oxidationsmittelzubereitung auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen. Hierfür geeignete Enzyme sind z. B. Peroxidasen, die die Wirkung geringer Mengen an Wasserstoffperoxid deutlich verstärken können. Ein Einsatz bestimmter Metallionen oder - komplexe kann ebenfalls bevorzugt sein. Geeignete Metallionen sind beispielsweise Zn²⁺, Cu²⁺, Fe²⁺, Fe³⁺, Mn²⁺, Mn⁴⁺, Li⁺, Mg²⁺, Ca²⁺, Ce⁴⁺, V³⁺, Co²⁺, Ru³⁺ und Al³⁺. Besonders geeignet sind dabei Zn²⁺, Cu²⁺ und Mn²⁺.

Weiterhin hat es sich als vorteilhaft erweisen, wenn die Oxidationsmittelzubereitungen mindestens einen Stabilisator oder Komplexbildner enthalten. Besonders bevorzugte Stabilisatoren sind Phenacetin, Alkalibenzoate (Natriumbenzoat) und Salicylsäure.

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mindestens "zweizähnig" ist. Gebräuchliche und im Rahmen der vorliegenden Erfindung bevorzugte Chelatkomplexbildner sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Auch komplexbildende Polymere, also Polymere, die entweder in der Hauptkette selbst oder seitenständig zu dieser funktionelle Gruppen tragen, die als Liganden wirken können und mit geeigneten Metall-atomen in der Regel unter Bildung von Chelat-Komplexen reagieren, sind erfindungsgemäß einsetzbar. Die Polymer-gebundenen Liganden der entstehenden MetallKomplexe können dabei aus nur einem Makromolekül stammen oder aber zu verschiedenen Polymerketten gehören. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/ oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Weitere, erfindungsgemäß einsetzbare Wirk-, Hilfs- und Zusatzstoffe sind beispielsweise nicht-ionische Polymere (wie Vinylpyrrolidinon/Vinylacrylat-Copolymere, Polyvinylpyrrolidinon und Vinylpyrrolidinon/Vinylacetat-Copolymere und Polysiloxane); zwitterionische und amphotere Polymere (wie Acrylamidopropyltrimethylammoniumchlorid/Acrylat-Copolymere und Octylacrylamid/Methylmethacrylat/tert-Butylaminoethylmethacrylat/2-Hydroxypropylmethacrylat-Copolymere); Verdickungsmittel (wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie Bentonit oder vollsynthetische Hydrokolloide wie z.B. Polyvinylalkohol); Strukturanden (wie Zucker, Maleinsäure und Milchsäure) und Konsistenzgeber (wie Zuckerester, Polyolester oder Polyolalkylether); Proteinhydrolysate (insbesondere Elastin-, Kollagen-, Keratin-, Milcheiweiß-, Sojaprotein- und Weizenproteinhydrolysate, deren Kondensationsprodukte mit Fettsäuren); Parfümöle; Cyclodextrine; Lösungsmittel und -vermittler (wie Ethanol, Isopropanol, Ethylenglykol, Propylenglykol, Glycerin, Dimethylisosorbid und Diethylenglykol); Entschäumer wie Silicone; Farbstoffe und Pigmente zum Anfärben des Mittels; Antischuppenwirkstoffe (wie Piroctone Olamine, Zink Omadine und Climbazol); Lichtschutzmittel (insbesondere derivatisierte Benzophenone, Zimtsäure-Derivate und Triazine); Wirkstoffe (wie Allantoin, Pyrrolidoncarbonsäuren, Cholesterin und deren Salze); weitere Fette und Wachse (wie Fettalkohole, Bienenwachs, Montanwachs und Paraffine); Quell- und Penetrationsstoffe (wie Glycerin, Propylenglykolmonoethylether, Carbonate, Hydrogencarbonate, Guanidine, Harnstoffe sowie primäre, sekundäre und tertiäre Phosphate); Trübungsmittel (wie Latex, Styrol/PVP- und Styrol/Acrylamid-Copolymere); Perlglanzmittel (wie Ethylenglykolmono- und -distearat sowie PEG-3-distearat); Treibmittel (wie Propan-Butan-Gemische, N₂O, Dimethylether, CO₂ und Luft) sowie Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die gebrauchsfertigen Mittel aus zweiphasigem Mittel (M1) und Farbveränderungsmittel (M2) weisen bevorzugt einen pH-Wert im Bereich von 6 bis 12 auf. Erfindungsgemäß bevorzugte Mittel sind dadurch gekennzeichnet, dass sie einen alkalischen pH-Wert besitzen. Eine weitere bevorzugte Ausführungsform der vorliegenden Erfindung besteht darin, dass das anwendungsbereite Mittel einen pH-Wert zwischen 7,0 und 12,0, bevorzugt zwischen 8,0 und 11,0 besitzt. Bei den pH-Werten im Sinne der vorliegenden Erfindung handelt es sich um pH-Werte, die bei einer Temperatur von 22 °C gemessen wurden.

Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Erfindungsgemäß ganz besonders bevorzugte Alkanolamine werden ausgewählt aus der Gruppe 2-Aminoethan-1-ol (Monoethanolamin), 2-Amino-2-methylpropan-1-ol und 2-Amino-2-methyl-propan-1,3-diol. Ein insbesondere bevorzugtes Alkanolamin ist Monoethanolamin. Geeignete basische Aminsäuren sind Lysin, Arginin und Ornithin. Das erfindungsgemäße, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat.

Die Anwendungstemperaturen können in einem Bereich zwischen 15 und 40 °C liegen. Nach einer Einwirkungszeit von 2 bis 60, bevorzugt 5 bis 45 Minuten wird das Blondiermittel durch Ausspülen von dem Haar entfernt. Das Nachwaschen mit einem Shampoo entfällt, wenn ein stark tensidhaltiger Träger verwendet wurde.

Weiterhin ist wesentlich, dass die Mittel (M1) und (M2) hinreichend flüssig sind, dass eine gute Vermischung untereinander möglich ist. Dazu ist vorteilhaft, wenn die Mittel nicht als Paste, zähe Creme oder eingedicktes Gel vorliegen, sondern über eine ausreichende Fließfähigkeit verfügen. Weiterhin müssen die anwendungsbereiten Mittel nach der Vermischung der Einzelkomponenten zwar über rheologische Eigenschaften verfügen, die ein Auftragen auf die zu färbenden Fasern erlauben, aber gleichzeitig ein Verlaufen oder Ausfließen des Mittels vom Wirkort während der Anwendungsdauer verhindern. Bevorzugt besitzen die Anwendungsmischungen daher eine Viskosität von 5 bis 100 Pa·s, bevorzugt von 10 bis 50 Pa·s, insbesondere von 10 bis 20 Pa·s und insbesondere bevorzugt von 10 bis 16 Pa·s (Brookfield, 22°C, Spindel #5, 4 rpm).

Je nach Zusammensetzung der Mittel (M1) und (M2) ist erfindungsgemäß bevorzugt, die Anwendungsmischungen erst unmittelbar vor der Anwendung durch Vermischen von Mittel (M1) und Mittel (M2) herzustellen. Dies ist insbesondere bei Inkompatibilitäten zwischen einzelnen Inhaltsstoffen vorteilhaft. Daher ist eine bevorzugte Darreichungsform des anwendungsbereiten Mittels eine getrennte Verpackungseinheit, worin die Mittel (M1) und (M2) jeweils getrennt voneinander verpackt vorliegen.

Ein weiterer Gegenstand der vorliegenden Erfindung ist daher eine Mehrkomponentenverpackungseinheit (Kit-of-Parts), welche mindestens zwei voneinander getrennt konfektionierte Container enthält, wobei ein erster Container (C1) ein kosmetisches Mittel (M1) gemäß dem ersten Erfindungsgegenstand enthält und ein zweiter Container (C2) eine Farbveränderungszubereitung (M2), enthaltend in einem kosmetischen Träger mindestens eine farbverändernde Komponente, enthält.

Unter Container wird im Rahmen der vorliegenden Erfindung eine Umhüllung verstanden, die in Form einer gegebenenfalls wieder-verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich jedoch dabei um Umhüllungen aus Glas oder Kunststoff. Besonders bevorzugt zur Visualisierung der zweiphasigen Mittel (M1) ist eine Ausführungsform, bei der die Umhüllung des Containers, welcher das Mittel (M1) enthält, für den Anwender durchsichtig ist. Eine bevorzugte Ausführungsform der erfindungsgemäßen Mehrkomponentenverpackungseinheit ist daher dadurch gekennzeichnet, dass der erste Container (C1), enthaltend das Mittel (M1), eine durchsichtige Verpackung, bevorzugt eine durchsichtige Kunststoffverpackung, besitzt.

Eine weitere Ausführungsform dieses Erfindungsgegenstands ist gegeben, wenn das Mittel (M2) aus Container (C2) die Färbezubereitung darstellt und als farbverändernde Komponente mindestens ein Oxidationsfarbstoffvorprodukt und/oder mindestens einen direktziehenden Farbstoff und/oder mindestens ein Aufhellmittel enthält. Eine Ausführungsform der vorliegenden Erfindung ist daher eine Mehrkomponentenverpackungseinheit dieses Erfindungsgegenstands, die dadurch gekennzeichnet ist, dass die Farbveränderungszubereitung (M2) als farbverändernde Komponente mindestens ein Oxidationsfarbstoffvorprodukt enthält.

Weiterhin kann es erfindungsgemäß besonders vorteilhaft sein, wenn das genannte Kit-of-Parts mindestens ein weiteres Haarbehandlungsmittel in einem getrennten Container enthält, insbesondere ein Konditioniermittel. Darüber hinaus kann die Verpackungseinheit Applikationshilfen, wie Kämme, Bürsten oder Pinsel, persönliche Schutzkleidung, insbesondere Ein-Weg-Handschuhe, sowie gegebenenfalls eine Gebrauchsanleitung umfassen.

Hinsichtlich der bevorzugten Ausführungsführungsformen der Mittel (M1) und (M2) gelten mutatis mutandis die obigen Ausführungen der vorangehenden Erfindungsgegenstände.

Bei der Anwendung der Mehrkomponentenverpackungseinheit kann es unerheblich sein, ob zunächst die beiden Phasen von Mittel (M1) durch kräftiges Schütteln kurzzeitig durchmischt werden und vor erneuter Phasentrennung mit dem Mittel (M2) vereinigt wird, um die anwendungsbereite Farbveränderungszubereitung bereitzustellen, oder ob zunächst das Mittel (M2) mit Mittel (M1) vereinigt wird und anschließend durch inniges Vermischen die anwendungsbereite Mischung hergestellt wird.

Ein weiterer Erfindungsgegenstand ist daher ein Verfahren zur Farbveränderung keratinischer Fasern, insbesondere menschlicher Haare, welches dadurch gekennzeichnet ist, dass aus einer Mehrkomponentenverpackungseinheit gemäß dem vorangegangenen Erfindungsgegenstand die beiden Mittel (M1) und (M2) in einem der Container (C2) oder (C1) vereinigt werden, der wiederverschlossene Container daraufhin geschüttelt wird, und das im Container resultierende, anwendungsbereite Farbveränderungsmittel anschließend auf die Fasern aufgebracht wird, für eine Einwirkdauer von 5 bis 60 min auf den Fasern belassen und schließlich ausgespült wird.

Zur verbesserten Durchmischung ist vorteilhaft, wenn der Container (C1), welcher das zweiphasige Mittel (M1) enthält, eine wieder-verschließbare Öffnung, wie beispielsweise einen Schnapp- oder einen Schraubverschluss, besitzt. Dies ermöglicht die erleichterte Zugabe des farbverändernden Mittels aus Container (C2), welcher seinerseits bevorzugt in Form eines Tütchens oder Sachets im Falle von wasserfreien, insbesondere pulverförmigen Farbveränderungsmitteln, oder in Form einer Tube im Falle von fließfähigen Farbveränderungsmitteln. Es ist bevorzugt, die einzelnen Zubereitungen zu vermischen und das anwendungsbereite Mittel zeitnah auf die keratinischen Fasern zu applizieren.

Eine besondere Ausführungsform dieses Erfindungsgegenstands ist daher ein Verfahren zur Farbveränderung keratinischer Fasern, insbesondere menschlicher Haare, welches dadurch gekennzeichnet ist, dass aus einer Mehrkomponentenverpackungseinheit gemäß dem vorangegangenen Erfindungsgegenstand der Inhalt des Containers (C2) in den Container (C1) gefüllt wird, der wiederverschlossene Container (C1) daraufhin geschüttelt wird, und das im Container (C1) resultierende, anwendungsbereite Farbveränderungsmittel anschließend auf die Fasern aufgebracht wird, für eine Einwirkdauer von 5 bis 60 min auf den Fasern belassen und dann ausgespült wird.

Ein weiterer Erfindungsgegenstand ist schließlich ein Verfahren zur Farbveränderung keratinischer Fasern, insbesondere menschlicher Haare, welches dadurch gekennzeichnet ist, dass aus einer Mehrkomponentenverpackungseinheit gemäß dem vorangegangenen Erfindungsgegenstand der Container (C1) geschüttelt wird, die entstandene Mischung der Phasen (I) und (II) unmittelbar anschließend mit einer Färbezubereitung des Containers (C2) innig vermischt, das resultierende, anwendungsbereite Farbveränderungsmittel anschließend auf die Fasern aufgebracht wird, für eine Einwirkdauer von 5 bis 60 min auf den Fasern belassen und schließlich ausgespült wird. Im Falle eines farbgebenden Mittels beträgt die bevorzugte Einwirkzeit 5 bis 40 min, bevorzugt 10 bis 30 min. Im Falle von aufhellenden oder bleichenden Farbveränderungsmitteln liegt die bevorzugte Einwirkzeit bei 30 bis 60 min, bevorzugt bei 40 bis 60 min.

Eine Ausführungsform des erfindungsgemäßen Verfahrens ist schließlich dadurch gekennzeichnet, dass das resultierende, anwendungsbereite Farbveränderungsmittel ein Viskosität von 10 bis 50 Pa·s, bevorzugt 10 bis 20 Pa·s und insbesondere bevorzugt 10 bis 16 Pa·s (Brookfield, 22°C, Spindel #5, 4 rpm), besitzt.

Im Rahmen dieses Gegenstandes der Erfindung gelten mutatis mutandis die oben getroffenen Aussagen analog.

### Beispiele

### 1) Färbecreme FC (Tabelle 1; Mengenangaben in Gew.-%)

| | |
|---|---|
| Lanette D | 6,60 |
| Lorol C12-18 techn. | 2,40 |
| Eumulgin B 2 | 0,60 |
| Eumulgin B 1 | 0,60 |
| Akypo Soft 45HP | 10,00 |
| Protelan MST 35 | 6,00 |
| Texapon K 14 S Special, 70 % | 2,80 |
| Produkt W 37194 | 3,75 |
| Natriumsulfit, wasserfrei | 0,00 |
| Ascorbinsäure | 0,10 |
| HEDP, wässrig, 60% | 0,20 |
| Natriumsilikat 40/42 | 0,50 |
| Kaliumhydroxid, wässrig, 50% | 1,00 |
| Glycin | 1,00 |
| Taurin | 1,00 |
| Alpha-Liponsäure | 0,20 |
| Litchiderm LS 9704 | 1,00 |
| p-Toluylendiaminsulfat | 2,81 |
| 2,4-Diaminophenoxyethanol 2HCl | 0,44 |
| Resorcin | 1,00 |
| m-Aminophenol | 0,20 |
| Monoethanolamin | 9,20 |
| Parfum | qs |
| Wasser, vollentsalzt | Ad 100 |

Rohstoffe: Lanette D (INCI-Bezeichnung: Cetearyl alcohol; Cognis); Lorol C12-18 techn. (INCI-Bezeichnung: Coconut alcohol; Cognis); Eumulgin B 2 (INCI-Bezeichnung: Ceteareth-20; Cognis); Eumulgin B 1 (INCI-Bezeichnung: Ceteareth-12; Cognis); Akypo Soft 45HP (ca. 21 %, INCI-Bezeichnung: Sodium Laureth-6 Carboxylate, Aqua; KAO); Protelan MST 35 (ca. 35 %, INCI-Bezeichnung: Sodium Myristoyl Sarconsinate, Sodium Methyl Cocoyl Taurate, Aqua; Zschimmer & Schwarz); Texapon K 14 S Special (ca. 70 %, INCI-Bezeichnung: Sodium Myreth Sulfate, Aqua; Cognis); Produkt W 37194 (ca. 20 %,INCI-Bezeichnung: Acrylamidopropyltrimonium Chloride / Acrylates Copolymer, Aqua; Stockhausen); Litchiderm LS 9704 (INCI-Bezeichnung: Butylene Glycol; Lichti Chinensis Pericarp Extract; Laboratoires Serobiologiques).

Die Fettbasis wurde zusammen bei 80 °C aufgeschmolzen und mit einem Teil der Wassermenge dispergiert. Anschließend wurden die restlichen Rezepturbestandteile unter Rühren der Reihe nach eingearbeitet. Es wurde mit Wasser auf 100 Gew.-% aufgefüllt und die Formulierung kalt gerührt.

### 2) Entwicklerzubereitungen EW (Tabelle 2; Mengenangaben in Gew.-%)

Folgende Entwicklerzubereitungen wurden mit unterschiedlichen polymeren Verdickungsmitteln sowie zwei unterschiedlichen hydrophoben Ölen hergestellt.

| **Rohstoff** | **E1** | **V1** | **V2** | **V3** | **V4** | **E2** | **V5** | **V6** | **V7** | **V8** |
|---|---|---|---|---|---|---|---|---|---|---|
| Natronlauge, 45 % techn. | 0,73 | 0,73 | 0,73 | 0,73 | 0,73 | 0,73 | 0,73 | 0,73 | 0,73 | 0,73 |
| Dipicolinsäure | 0,10 | 0,10 | 0,10 | 0,10 | 0,10 | 0,10 | 0,10 | 0,10 | 0,10 | 0,10 |
| Dinatriumpyrophosphat | 0,03 | 0,03 | 0,03 | 0,03 | 0,03 | 0,03 | 0,03 | 0,03 | 0,03 | 0,03 |
| HEDP, wässrig, 60 Gew.-% | 1,50 | 1,50 | 1,50 | 1,50 | 1,50 | 1,50 | 1,50 | 1,50 | 1,50 | 1,50 |
| Texapon NSO | 2,00 | 2,00 | 2,00 | 2,00 | 2,00 | 2,00 | 2,00 | 2,00 | 2,00 | 2,00 |
| Dow Corning DB 110 A | 0,07 | 0,07 | 0,07 | 0,07 | 0,07 | 0,07 | 0,07 | 0,07 | 0,07 | 0,07 |
| Carbomer 954, wässrig, 4 Gew.-% | 36,00 | -- | -- | -- | -- | 36,00 | -- | -- | -- | -- |
| Aculyn 33A | -- | -- | 15,00 | -- | -- | -- | -- | 15,00 | -- | -- |
| Xanthan, wässrig, 4 Gew.-% | -- | -- | -- | 36,00 | -- | -- | -- | -- | 36,00 | -- |
| Natrosol 250 % HHX, wässrig, 4 Gew.-% | -- | -- | -- | -- | 36,00 | -- | -- | -- | -- | 36,00 |
| Wasserstoffperoxid, wässrig, 50 Gew.-% | 12,00 | 12,00 | 12,00 | 12,00 | 12,00 | 12,00 | 12,00 | 12,00 | 12,00 | 12,00 |
| flüssiges Paraffin | 16,66 | 16,66 | 16,66 | 16,66 | 16,66 | -- | -- | -- | -- | -- |
| Isopropylmyristat | -- | -- | -- | -- | -- | 16,66 | 16,66 | 16,66 | 16,66 | 16,66 |
| Wasser, vollentsalzt | Ad 100 | | | | | | | | | |

Rohstoffe: Texapon NSO (ca. 27%, INCI-Bezeichnung: Sodium Laureth Sulfate; Cognis); Dow Corning DB 110 A (INCI-Bezeichnung: Dimethicon; Dow Corning); Carbomer 954 (INCI-Bezeichnung: Carbomer; Noveon); Aculyn 33A (ca. 28%; INCI-Bezeichnung: Acrylates Copolymer, Aqua; Rohm & Haas); Natrosol 250 HHX (INCI-Bezeichnung: Hydroxyethylcellulose; Hercules).

Zur Herstellung der Entwicklerzubereitungen wurden die Rohstoffe mit Ausnahme der Öle (Paraffinöl sowie Isopropylmyristat) vorgemischt. Anschließend wurden die Öle zugegeben. Tabelle 3 zeigt die Beschaffenheit der erhaltenen Mittel:

| **Tabelle 3** | **E1** | **V1** | **V2** | **V3** | **V4** | **E2** | **V5** | **V6** | **V7** | **V8** |
|---|---|---|---|---|---|---|---|---|---|---|
| Beschaffenheit vor Ölzugabe | flüssig | | | pastös | festes Gel | flüssig | | | pastös | festes Gel |
| Phasentrennung ? | 2-phasig | | | emulgiert | | 2-phasig | | | emulgiert | |
| Beschaffenheit der hydrophoben Phase nach Ölzugabe* | + | + | 0 | -- | -- | + | + | 0 | -- | -- |

| | | | | | | | | | | |
|---|---|---|---|---|---|---|---|---|---|---|
| *Symbole: "+" : klare, transparente Ölphase; "0" : Ölphase mit Eintrübungen an der Phasengrenzfläche; "--" : keine separate Ölphase | | | | | | | | | | |

### 3) Anwendungsmischungen:

Die Färbecreme FC wurde vor der Anwendung jeweils mit den Entwicklerlösungen bei Raumtemperatur im Gewichtsverhältnis von 1:1 versetzt und innig vermischt.

Dabei wurden folgende Mischviskositäten erhalten (Tabelle 4):

| **Färbemittel** | **Anwendungsmischung** | **Mischviskosität* [mPa·s]** |
|---|---|---|
| #1 | FC + E1 (erfindungsgemäß) | 14200 |
| #2 | FC + V1 (nicht erfindungsgemäß) | 2900 |
| #3 | FC + V2 (nicht erfindungsgemäß) | 20100 |
| #4 | FC + V3 (nicht erfindungsgemäß) | 30600 |
| #5 | FC + V4 (nicht erfindungsgemäß) | 33000 |
| #6 | FC + E2 (erfindungsgemäß) | 14600 |
| #7 | FC + V5 (nicht erfindungsgemäß) | 3500 |
| #8 | FC + V6 (nicht erfindungsgemäß) | 19900 |
| #9 | FC + V7 (nicht erfindungsgemäß) | 29900 |
| #10 | FC + V8 (nicht erfindungsgemäß) | 17900 |

| | | |
|---|---|---|
| * gemessen mit einem Brookfield-Viskosimeter DV-II+Pro mit Spindel #5 bei 4 Upm bei RT (22°C) in 590 mL Bechergläser, hohe Form. | | |

### 4) Ergebnisse

Die Oxidationszubereitungen V3 und V4 sowie V7 und V8 wiesen keine geeignete Viskosität für eine gute Durchmischung mit der Färbezubereitung auf (Tabelle 3). Außerdem bildeten diese Entwicklerzubereitungen nach Ölzugabe keine zwei getrennten Phasen aus. Die nicht-erfindungsgemäßen Oxidationszubereitungen V2 und V6 zeigten zwar zwei getrennte Phase, jedoch wiesen die hydrophoben Ölphasen jeweils Eintrübungen durch Mikroemulsionen an der Grenzfläche auf und sind daher für kosmetische Anwendungen ungeeignet. Die nicht erfindungsgemäßen Oxidationszubereitungen V1 und V5 besitzen zwar die erwünschten zwei Phasen mit klarer Ölphase, jedoch sind die Mischviskositäten der Anwendungsmischungen zu gering (#2 und #7; Tabelle 4).

Lediglich die erfindungsgemäßen Oxidationszubereitungen E1 und E2 besitzen zwei klar getrennte Phasen mit klarer Ölphase und ergeben nach Vermischen mit der Färbecreme anwendungsbereite Färbemittel (#1 und #6) mit gewünschter Viskosität für die Anwendung auf menschlichen Haaren.

## Patentansprüche

1. Kosmetisches Mittel für die Behandlung keratinischer Fasern, **dadurch gekennzeichnet, dass** es zwei nebeneinander vorliegende, aber durch eine Phasengrenze voneinander getrennte Phasen umfasst, die in zwei Schichten übereinander mit unmittelbarem Kontakt über eine gemeinsame Grenzfläche zueinander vorliegen,
wobei die erste Phase (I) eine wässrige Phase darstellt, die Wasserstoffperoxid und mindestens ein anionisches, polymeres Verdickungsmittel, ausgewählt aus vernetzten oder unvernetzten Polyacrylsäure-Polymeren enthält, und
wobei die zweite Phase (II) eine klare, hydrophobe Ölphase darstellt, die als Öl mindestens einen flüssigen Carbonsäureester aus C₂-C₈-Monoalkanol mit einer Mono- oder Dicarbonsäure und/oder mindestens ein Paraffinöl enthält, **dadurch gekennzeichnet, dass**
das anionische polymere Verdickungsmittel ausgewählt ist aus Polyacrylsäuren, die ein Molekulargewicht MW von mindestens 500000 g/mol besitzen,
das Mittel nichtionische, anionische, zwitterionische und/oder amphotere Tenside und/oder Emulgatoren in einem Gesamtgewicht von weniger als 1 Gew.-%, jeweils bezogen auf das Gesamtgewicht des Mittels, enthält und
das Öl der klaren, hydrophoben Ölphase (II) ausgewählt ist aus der Gruppe, die gebildet wird aus Isopropylpalmitat, Isopropylmyristat, Dibutyladipat und flüssigem Paraffinöl.

2. Mehrkomponentenverpackungseinheit (Kit-of-Parts), enthaltend mindestens zwei voneinander getrennt konfektionierte Container, wobei ein erster Container (C1) eine kosmetische Zubereitung (M1) gemäß Anspruch 1 enthält und ein zweiter Container (C2) eine Farbveränderungszubereitung (M2), enthaltend in einem kosmetischen Träger mindestens eine farbverändernde Komponente, enthält.

3. Mehrkomponentenverpackungseinheit gemäß Anspruch 2, **dadurch gekennzeichnet, dass** der erste Container (C1) eine durchsichtige Verpackung, bevorzugt eine durchsichtige Kunststoffverpackung, besitzt.

4. Mehrkomponentenverpackungseinheit gemäß einem der Ansprüche 2 oder 3, **dadurch gekennzeichnet, dass** die Farbveränderungszubereitung (M2) als farbverändernde Komponente mindestens ein Oxidationsfarbstoffvorprodukt enthält.

5. Verfahren zur Farbveränderung keratinischer Fasern, insbesondere menschlicher Haare, **dadurch gekennzeichnet, dass** aus einer Mehrkomponentenverpackungseinheit gemäß einer Ansprüche 2 bis 4 die beiden Mittel (M1) und (M2) in einem der Container (C2) oder (C1) vereinigt werden, der wiederverschlossene Container daraufhin geschüttelt wird, und das im Container resultierende, anwendungsbereite Farbveränderungsmittel anschließend auf die Fasern aufgebracht wird, für eine Einwirkdauer von 5 bis 60 min auf den Fasern belassen und schließlich ausgespült wird.

6. Verfahren nach Anspruch 5, **dadurch gekennzeichnet, dass** das resultierende, anwendungsbereite Farbveränderungsmittel ein Viskosität von 10 bis 50 Pa·s, bevorzugt 10 bis 20 Pa·s (Brookfield, 22°C, Spindel #5, 4 rpm), besitzt.

## Claims

1. A cosmetic agent for treating keratin fibers, **characterized in that** it comprises two phases that are beside one another but separated from one another by a phase boundary and are provided in two layers one on top of the other so as to be in direct contact at a shared boundary surface,
the first phase (I) being an aqueous phase which contains hydrogen peroxide and at least one anionic polymer thickener, selected from crosslinked or non-crosslinked polyacrylic acid polymers, and
the second phase (II) being a clear, hydrophobic oil phase which contains, as the oil, at least one liquid carboxylic acid ester of C₂-C₈ monoalkanol having a mono- or dicarboxylic acid and/or at least one paraffin oil, **characterized in that**
the anionic polymer thickener is selected from polyacrylic acids which have a molecular weight MW of at least 500,000 g/mol,
the agent contains non-ionic, anionic, zwitterionic and/or amphoteric surfactants and/or emulsifiers in a total weight of less than 1 wt.% based on the total weight of the agent in each case, and
the oil in the clear, hydrophobic oil phase (II) is selected from the group formed by isopropyl palmitate, isopropyl myristate, dibutyl adipate and liquid paraffin oil.

2. A kit of parts, containing at least two separately packaged containers, wherein a first container (C1) contains a cosmetic agent (M1) according to claim 1 and a second container (C2) contains a color-changing preparation (M2) containing at least one color-changing component in a cosmetic carrier.

3. The kit of parts according to claim 2, **characterized in that** the first container (C1) has transparent packaging, preferably transparent plastics packaging,

4. The kit of parts according to one of claims 2 or 3, **characterized in that** the color-changing preparation (M2) contains at least one oxidation dye precursor as the color-changing component.

5. A method for changing the color of keratin fibers, in particular human hair, **characterized in that**, from a kit of parts according to one of claims 2 to 4, the two agents (M1) and (M2) are combined in one of the containers (C2) or (C1), the container (C2) is closed again and then shaken, and the resulting ready-to-use color-changing agent in the container is then applied to the fibers, left on the fibers for a reaction time of from 5 to 60 minutes and then rinsed out.

6. The method according to claim 5, **characterized in that** the resulting ready-to-use color-changing agent has a viscosity of from 10 to 50 Pa·s, preferably from 10 to 20 Pa·s (Brookfield, 22°C, spindle #5, 4 rpm).

## Revendications

1. Produit cosmétique pour le traitement de fibres de kératine, **caractérisé en ce qu'**il comprend deux phases juxtaposées mais séparées l'une de l'autre par une interface, qui sont présentes en deux couches l'une sur l'autre, avec un contact immédiat par une surface limite commune,
la première phase (I) représentant une phase aqueuse qui contient du peroxyde d'hydrogène et au moins un épaississant polymère anionique choisi parmi des polymères de polyacide acrylique réticulé ou non, et
la seconde phase (II) représentant une phase organique hydrophobe claire, qui contient comme huile au moins un ester d'acide carboxylique liquide d'un C₂₋₅-monoalcool avec un acide mono- ou dicarboxylique et/ou une paraffine,
**caractérisé en ce que** l'épaississant polymère anionique est choisi parmi des polyacides acryliques ayant un poids moléculaire MW d'au moins 500 000 g/mol, le produit contient des tensioactifs et/ou des émulsifiants non-ioniques, anioniques, zwitterioniques et/ou amphotères à hauteur d'un poids total inférieur à 1 % en poids rapporté au poids total du produit, et l'huile de la phase organique claire hydrophobe (II) est choisie dans le groupe constitué du palmitate d'isopropylo, du myristate d'isopropyle, de l'adipate de dibutyle et d'une huile de paraffine liquide.

2. Unité d'emballage multicomposant (kit) contenant au moins deux récipients séparés l'un de l'autre, un premier récipient (C1) contenant une préparation cosmétique (M1) selon la revendication 1 et un second récipient (C2) contenant une préparation colorante (M2) contenant, dans un vecteur cosmétique, au moins un composant modificateur de couleur.

3. Unité d'emballage multicomposant selon la revendication 2, **caractérisé en ce que** le premier récipient (C1) possède un emballage transparent, de préférence un emballage en plastique transparent.

4. Unité d'emballage multicomposant selon une des revendications 2 ou 3, **caractérisé en ce que** la préparation colorante (M2) contient comme composant modificateur de couleur au moins un précurseur de coloration d'oxydation.

5. Procédé de modification de la couleur de fibres de kératine, en particulier de cheveux humains, **caractérisé en ce qu'**à partir d'une unité d'emballage multicomposant selon une des revendications 2 à 4, on réunit les deux produits (M1) et (M2) dans un des récipients (C2) ou (C1), puis on secoue le récipient refermé, et on applique sur les fibres le produit modificateur de couleur prêt à l'emploi qui en résulte dans le récipient, on le laisse agir sur les fibres pendant une durée d'action de 5 à 60 minutes, enfin on rince.

6. Procédé selon la revendication 5, **caractérisé en ce que** le produit résultant modificateur de couleur prêt à l'emploi possède une viscosité de 10 à 50 Pa.s, de préférence de 10 à 20 Pa.s (Brookfield, 22°C, rotor n°5, 4 tr/min).
